# EUROPEAN PATENT SPECIFICATION

(11) **EP 3 289 351 B1**
(45) Date of publication and mention of the grant of the patent: **01.11.2023**
(21) Application number: 16789347.8
(22) Date of filing: 28.04.2016
(51) Int. Cl.: G01N 33/12, G01N 33/483, G01N 29/02, G01N 29/06, G01N 29/24, G01N 29/44, G01N 33/487

(54) **DEVICE WITH A MICRO OR NANO MECHNICAL BASED SENSOR FOR DETECTION OF DECOMPOSITION MOLECULES LIKE BIOGENIC AMINES (ASSOCIATED WITH FOOD SPOILAGE AND CERTAIN HUMAN DISEASES AMONG OTHERS) AND SUBSEQUENT COMPUTATION FOR DETERMINATION OF FRESHNESS AND EXPIRATION DATE**
VORRICHTUNG MIT EINEM MIKRO- ODER NANOMECHANISCHEM SENSOR ZUM NACHWEIS VON ZERSETZUNGSMOLEKÜLEN WIE BIOGENEN AMINEN (DIE UNTER ANDEREM MIT LEBENSMITTELVERDERBLICHKEIT UND BESTIMMTEN MENSCHLICHEN KRANKHEITEN ASSOZIIERT SIND) UND ANSCHLIESSENDER BERECHNUNG ZUR BESTIMMUNG DER FRISCHE UND DES VERFALLSDATUMS
DISPOSITIF MUNI D'UN CAPTEUR BASÉ SUR LA MICROMÉCANIQUE OU LA NANOMÉCANIQUE POUR LA DÉTECTION DE MOLÉCULES DE DÉCOMPOSITION TELLES QUE DES AMINES BIOGÈNES (ASSOCIÉES À LA DÉTÉRIORATION DES ALIMENTS ET CERTAINES MALADIES HUMAINES ENTRE AUTRES) ET CALCUL CONSÉCUTIF POUR DÉTERMINER LA FRAÎCHEUR ET LA DATE D'EXPIRATION

(30) Priority: 01.05.2015 DK 201500266
(43) Date of publication of application: 07.03.2018
(73) Proprietor: AmiNIC ApS, 5500 Middelfart (DK)
(72) Inventor: HVAM, Jeanette, 5000 Odense C (DK); ROBERTSON, Elizabeth, 413 28 Göteborg (SE); HAMMER, Runi, 5250 Odense SV (DK)
(74) Representative: Plougmann Vingtoft a/s
(86) International application number: PCT/DK2016/050110
(87) International publication number: WO 2016/177374

(56) References cited:
- US-A1- 2005 074 817
- US-A1- 2008 299 669
- US-A1- 2009 084 162
- US-A1- 2011 086 368
- US-B1- 6 631 333
- PRABHA VERMA ET AL: "Polymer selection for SAW sensor array based electronic noses by fuzzy c-means clusteriing of partition coefficients: Model studies on detection of freshness and spoilage of milk and fish.", SENSORS AND ACTUATORS B: CHEMICAL, vol. 209, no. 31, 31 March 2015 (2015-03-31), pages 751-769, XP002784256,
- E. OHASHI ET AL: "Sensors for the food industry", FOOD CONTROL, vol. 4, no. 4, 1 January 1993 (1993-01-01) , pages 183-188, XP55022231, ISSN: 0956-7135, DOI: 10.1016/0956-7135(93)90248-M
- GIANNAKOUROU M C ET AL: "Field evaluation of the application of time temperature integrators for monitoring fish quality in the chill chain", INTERNATIONAL JOURNAL OF FOOD MICROBIOLOGY, ELSEVIER BV, NL, vol. 102, no. 3, 25 July 2005 (2005-07-25) , pages 323-336, XP027663146, ISSN: 0168-1605 [retrieved on 2005-07-25]
- SOFU A ET AL: "Estimation of Storage Time of Yogurt with Artificial Neural Network Modeling", JOURNAL OF DAIRY SCIENCE, AMERICAN DAIRY SCIENCE ASSOCIATION, US, vol. 90, no. 7, 1 July 2007 (2007-07-01), pages 3118-3125, XP026956040, ISSN: 0022-0302 [retrieved on 2007-07-01]
- M. Horsfall Jnr* ET AL: "EVALUATION OF THE LEVELS OF TOTAL VOLATILE BASES AND TRIMETHYLEAMINE FORMED IN FISH STORED AT LOW TEMPERATURE", BULLETIN OF THE CHEMICAL SOCIETY OF ETHIOPIA, vol. 20, no. 1, 8 May 2006 (2006-05-08), XP055685982, ET ISSN: 1011-3924, DOI: 10.4314/bcse.v20i1.21155

## Description

### Field of invention

The current invention relates to a micro- or nano-mechanical sensing device e.g. a micro or nano cantilever functionalized with a chemical e.g. a molecular imprinted polymer (MIP), which selectively binds biogenic amines such as cadaverine and/or putrescine. The invention also covers the method of converting the mechanical change in the cantilever into an electrical signal and digital value related to, for example, the decomposition state of the meat. It also includes the different utilizations and applications of this specific technology in monitoring food spoilage and human disease indicators. The invention also relates to the subsequent digital handling and computation of the measured values. The values are cross-referenced with a database containing algorithms for determining the decomposition rate of the meat sample in question and thus provide the exact degradation state and forecast a correct expiration of the meat sample.

### Background of the invention

The development of sensitive and specific detection devices with applications in biochemistry and molecular biology has evolved considerably in recent years. *In situ* measurements are in high demand and are under continuous investigation and improvement. In particular, considerable research effort has been invested in the detection and quantification of biomarkers related to human disease and food quality.

Traditionally, meat and fish quality is determined by sight, smell and touch, where trained individuals evaluate the food quality. However, this remains a highly subjective assessment and can only be performed by correctly trained and experienced personnel.

For an untrained individual it can be extremely difficult to distinguish between products which are close to the limit between 'pass' or 'fail'. In addition laboratory tests of food quality are often both costly and time consuming, despite the fact that the ability to assess food quality and spoilage quickly, cheaply and easily is in increasing demand. Forecasting of shelf-life has previously relied on the condition that the cooling chain has not been breach and does not take into consideration the historic of a specific meat sample.

Spoilage of food such as meat and fish is a result of unconstrained bacterial growth once the animal's immune and circulatory system is terminated at the time of slaughter. Most bacterial flora in meat and fish products has no direct influence on the degradation state. As such, direct detection of bacteria is not suitable for determining the degradation state of the product and can thus not be associated with spoilage in general. However, a pronounced degradation marker is the decarboxylation of amino acids by enzymes originating from microorganisms in and on the meat. These biomarkers are biogenic amines, such as tyramine, histamine, putrescine and cadaverine. Of these, the latter two are volatile diamines with very distinctive odours and thus are present in the gas phase under normal conditions. Furthermore, the concentration of these correlate well with the relative numbers of spoilage bacterial; for fresh meats correlating directly to the total viable count (TVC). Thus these volatile primary diamines are frequently used as indicators of freshness both by trained personal and by private consumers. Many foodborne illnesses are directly related to the toxicity of biogenic amines that are not eliminated with heat treatment of the food. In addition, the TVC of bacteria in the food is in turn related to the concentration of pathogenic bacteria. In this way, the degradation of meats and fish/seafood during storage not only reduces quality but also introduces significant risks to human health and to the economy potentially generated from the sale of meat products.

Technological innovations are key to the maintenance of food quality in modern society. However, very few sensors are small, fast and inexpensive enough to be used for *in situ* measurements. Typically, gas detection involves time consuming and expensive techniques such as, for biogenic amines, an initial isolation of the amine and subsequent use of either gas-chromatography (GC), mass spectroscopy (MS) or high performance liquid chromatography (HPLC). These methods are inherently large and immobile (often only available at specialised institutes and universities) and measurements come at a high cost. In these methods, following the isolation and extraction of the targeted amines, chemical composition is determined and finally quantified and analysed. In some cases, rapid and more coarse scanning is sufficient in regard to food quality and biomedical applications; however, there is a need for a sensing device that simply, accurately, rapidly and inexpensively can determine the presence of biogenic amines.

Biogenic amine concentration can be utilised to indicate the freshness of food such as meat, fish, cheese, alcoholic beverages and other fermented food. Additionally, amines such as aniline and toluidine among other biogenic amines have been associated with a variety of human diseases. Therefore in addition the sensor or sensor array described in this invention can be used to determine concentrations of other amines in - for example - human urine, sweat, saliva, or other body fluids for biogenic amines acting as precursors for a variety of diseases and conditions. This is in addition to the sensor or sensor array being able to monitor food quality and thus ensure a reliable and secure shelf life by interacting with a database containing algorithms for determination of degradation rates, etc. In this way, this invention benefits both the consumer, the retail outlet as well as the meat distribution, transportation, pre-processing and production sites, and the environment; reducing the monetary cost of food waste in private businesses and households and reducing the carbon emissions associated with excess food production. This device may also be likely to have beneficial applications in the field of environmental monitoring and medicine.

Different types of meat - i.e. beef, pork, chicken, salmon, tuna, etc. - develop biogenic amines like cadaverine at different rates as they degrade, this rate is also affected by the storage conditions pork stored at 5 degrees' Celsius degenerates at a lower rate than pork stored at 22 degrees Celsius and beef will experience yet another cadaverine development as it ages). These concentrations of cadaverine, for different kinds of meat at different storage conditions, can be pre-determined by measurement and compiled into a database. The database thus consists of algorithms reflecting the concentration of cadaverine for several types of meat under several types of storage conditions. Since the concentration of cadaverine is an indicator of the degradation state and thus freshness of the meat and fish, one can by comparing the measured value for one specific meat sample to the algorithms precisely forecast the correct expiration date.

The patents US20060075803, WO2005119233, WO2004081572, WO2004074834, US2004080319, US657520, WO9938007 and WO2008035220 among others all describe cantilever based sensors with or without MIP for bio molecule detection, though none of these are specifically aimed at detecting biogenic amines.

The patents US2013011364, WO2009026581, US2008153079, WO2004027412, WO0177667 and a paper on Curcurbituril7 (Silva, Choudhury et. al., ACS Chem. Biol., 2014, 9, 1432-1436), describes selective binding of biogenic amines to e.g. MIP's. These compounds can be used to identify and bind specific biogenic amines, but the patents do not describe the application of this in a micro or nano mechanical device.

US2007224689, JPH01119752, US2011306140, PA05007224 and WO2006078255 disclose non-mechanical sensors for biogenic amine detection. These sensors detect cadaverine but either by electron conduction or chromatographically.

WO0078204, WO9966304, WO0054237 and US2002142477 disclose sensor arrays and remote computation of odours. These patents cover the concept of electronic noses consisting of multiple sensors as an array and the possibility to use the compilation of measured values at a remote location. These patents are not related to any sensing technique or target molecule specifically.

Prabha Verma et al: "Polymer selection for SAW sensor array based electronic noses by fuzzy c-means clustering of partition coefficients: Model studies on detection of freshness and spoilage of milk and fish", Sensors and Actuators B: Chemical, Vol. 209, no. 31, 31 March 2015 (2015-03-31), pages 751-769 discloses a device for determining freshness for fish, the device comprising one sensing device for detection of biogenic amines.

M. Horstfall Jnr* et al: "Evaluation of the levels of total volatile bases and trimethyleamine formed in fish stored at low temperature", Bulletin of the Chemical Society of Ethiopia, Vol. 20, no. 1, 8 May 2006 (2006-05-08), XP055685982, Et ISSN: 1011-3924, DOI: 10.4314/bcse.v20i1.21155 discloses a correlation between the concentration of TMA and storage time, and suggests to use the data obtained experimentally for formulating appropriate food safety limits for consumption of refrigerated fresh fish products.

### Object of the Invention

A first object of the invention is to achieve a sensor based on micro or nano mechanics - i.e. cantilever technology and can be utilized to offer a reliable handheld device for detection of trace amounts of volatile amines such as cadaverine and putrescine

A second object of the invention is to determining degradation states and expiration dates by correlating measured values of biogenic amine concentration to excising pre-measured values for different types of meat, fish and poultry, at different storage conditions; temperature and atmosphere composition.

A third object of the invention is that pre-measured values are stored in a database and constitutes algorithms to which the specific *in situ* measurement can be compared. The *in situ* measured value reflecting i.e. cadaverine concentration will be compared to values in the database from which the degradation state can be determined.

### Summary of the invention

A sensor based on micro or nano mechanics - i.e. cantilever technology and can be utilized to offer a reliable handheld device for detection of trace amounts of volatile amines such as cadaverine and putrescine. These compounds are known to be indicative of food spoilage, however this device may also be adapted to detect human disease precursors, or indicators of air and water pollution. In addition, the micro cantilever sensor technology offers a handheld, real-time sensor for chemicals, either in gaseous or in liquid phase, with high sensitivity, miniature size, low power consumption, and the ability to be fabricate into a sensor array for simultaneous detection of multiple chemicals. The chemical concentration will correspond to a chemical fingerprint. The measured values can be computed *in situ* for determining the state of degradation for the specific meat sample and forecast correct expiration date for further computation and remote access. Accordingly, it would be desirable to have, for example, a handheld, in-line or real-time monitoring micro- or nano-cantilever device for detection and identification of trace amounts of biogenic amines indicative of, for example, both food spoilage or human diseases.

### Brief description of the drawings

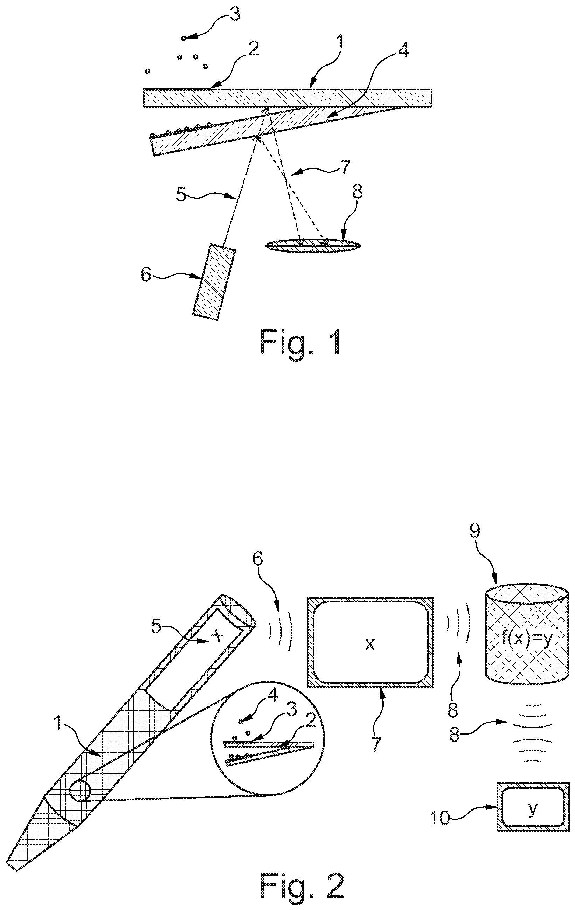

Fig. 1: Depicted is an example of a micro or nano mechanical sensor in this case a cantilever (1). The cantilever is coated by a selective binder e.g. a molecular imprinted polymer (2) that selectively captures the target molecule (3) in this case volatile biogenic amines. The cantilever is deformed as the molecules are attached to its surface (4), this mechanical change can be detected here optically; an incident laser beam (5) originating from a laser diode (6) will be deflected differently (7) and the difference can be detected with a photodiode (8).
Fig. 2: The drawing is a schematic representation of a device/apparatus in the form of a pen (1) that can be inserted into the sample of interest e.g. meat. The volatile biogenic amine concentration in the meat directly correlates to the concentration of these molecules in an equilibrium volume above the meat where the sensor e.g. a nano cantilever (2) is positioned. The selective binder coating on the cantilever e.g. a molecular imprinted polymer (3), selectively captures the target molecule (4), in this case biogenic amines like cadaverine. The bending correlates to a concentration which in turn correlates to e.g. a state of degradation of the meat. In a device related to but not forming part of the invention, this could be displayed on a display on the pen itself (5) and wirelessly transmitted (6) to a local computer (7). In such a device, an algorithm can here process the measured value to yield a correct expiration date for the meat and/or forwarded via the internet (8) to a remote location e.g. a database (9). Here the values will be stored and processed for later use and for remote asses from a computer or other similar device (10) via the internet (8).

### Detailed description of invention

### Technology:

The present invention is categorized as a BioMEMS or BioNEMS (Bio MicroElectroMechanical Systems/ Bio NanoElectroMechanical Systems), more specifically a miniaturized biosensor. The invention constitutes the combination of known technologies in order to measure low levels of biogenic amines as indicator of i.e. food spoilage and from this predict correct expiration dates of a specific meat, fish or poultry sample.

One embodiment of the invention relates to determining degradation states and expiration dates by correlating measured values of biogenic amine concentration to excising pre-measured values for different types of meat, fish and poultry, at different storage conditions; temperature and atmosphere composition. The pre-measured values are stored in a database and constitute algorithms to which the specific *in situ* measurement can be compared. The *in situ* measured value reflecting i.e. cadaverine concentration will be compared to values in the database from which the degradation state can be determined. As the user supplies information on storage conditions this measured value can through algorithms be utilized to correctly and precisely predict remaining shelf-life of the specific meat sample.

Another embodiment of the invention relates to a nano- or micro-mechanical sensor such as a stress or mass sensor for detection of target substances in fluids comprising one or more sensor units - e.g. shaped as bridges, diaphragms or cantilevers with capture coatings. Cantilevers are a method for sensing label-free parameters from induced stress and bending when in contact with the target molecule in the form of a fluid (gas or liquid) are well known in the arts. Micro- and nano-cantilevers are known for their high sensitivity and are well known for their use in detection via molecular interaction. They are micro-fabricated by e.g. etching, casting, nano-imprinting or soft lithography from materials such as silicon, silicon nitride, silicon carbide or polymer material. At least one face of the cantilever of any shape will be covered by a selective binding coating. This binding coating interacts either physically or chemically with the target molecule and induces a stress at the cantilever surface, heat generation or mass load. These parameters will physically alter the cantilever and can thus be detected as a direct correlation to the concentration of the chemical of interest/target molecule via prior calibration. A bending or change in Eigen frequency of the cantilever can be detected either optically or via a piezo resistor. Electrical detection can be obtained through a piezo resistor placed at the clamped end of the cantilever. Optical detection involves focusing the light from a laser diode onto the cantilever and measuring the change in deflection angle as the cantilever undergoes physical change. Both readout methods for detection of the mechanical change of the cantilever are known for a person skilled in the art.

Increasing interest in catalysts and selective binders as well as molecular imprinting polymer technology has revolutionized the surface sensitive sensors. A selective binder coating is essential in almost all fluid sensors and especially for odour sensors/electronic noses. The high affinity selective binder coating is required which should have a high selectivity towards the target molecule - in this case the specific biogenic amine, e.g. 1,4,8,11-Tetraazacyclotetradecane (cyclam) or molecular imprinting polymer technology offers this high target specific selectivity. Molecular imprinting essentially involves making a cast of a target molecule. This is achieved by letting monomers self-assemble around the target molecule and removing the target molecule leaving a complementary shape and functionality to the target molecule, and will thus only bind the original target molecule. The binding by the polymer of the target molecule is now highly selective and reversible. One specifically designed colour changing molecular imprinted polymer with a high selectivity towards cadaverine has already been patented and utilized for chromatographic detection purposes.

Thus in accordance with this embodiment, a method of detecting biogenic amines as indicator of food spoilage and as precursor for certain human diseases is disclosed. The technology entails a micro- or nano-mechanical device for label-free detection of the desired chemical. A selective binder coating with a high affinity towards biogenic amines will ensure selective binding and the mechanical change of the sensor can be detected either optically or electrically.

### Utilization and application:

An additional embodiment of the invention is the utilization of the technology as described in the first embodiment of the invention. The micro-or nano-mechanical sensor for detection of biogenic amines can be subjected to the fluid of interest by e.g. liquid exposure or gas exposure. This could be accomplished by e.g. integrating the technology in a microfluidic device, by direct contact with the fluid in the headspace of the substance of interest e.g. in the case of food spoilage, the meat or fish/seafood, or in the case of human diseases, breath, sweat or urine. In this way, concentrations of volatile biogenic amines can be measured as equilibrium concentrations in the headspace of the sample. Thus this embodiment of the present invention provides for non-destructive and label-free detection of the quality of food such as meat and seafood freshness at any given time and the determination of the remaining usable shelf life by comparison of measured values with pre-determined algorithms is thus disclosed.

In accordance with a further embodiment, an apparatus e.g. handheld, inline or real-time device wherein the described micro or nano mechanical technology for detection of biogenic amines is incorporated is disclosed. Such an apparatus would additionally be suitable for example in production, distribution, transportation, retail, industrial kitchens, hospitals, restaurants and in the private homes and research. It may additionally be used as an apparatus for inline measurement at production facilities handling meat or fish or as part of a test-kit or included in food packages. Utilized for medical applications such as for determining the presence of biogenic amines in body fluids as precursor of specific diseases, the technology described in the previous embodiment could additionally be applied for real-time monitoring of the progression in biogenic amine concentration whenever needed.

In yet another device related to but not forming part of the invention, the ability to process and send the measured values to a computer or a remote server may be used for e.g. further characterization, remote access, the ability to mine data from a database containing a plurality of chemical fingerprints. It may also be applied as a method for remote characterization, monitoring trends across populations or changes in a physical state of a fluid over a period of time detected by the technology described in the first embodiment. These remote databases may also contain the algorithms applied for determining exact degradation state and predict precise expiration dates as described in the first embodiment.

Accordingly, a remote detection system of such a device includes at least one sensor and includes numerous sensors, a measuring device for detecting a signal at the sensors, a device for transmitting the signal data to a remote location, a computer, a data structure of sensor response profiles or fingerprints, and a comparison algorithm.

Particular embodiments of the present invention have been described for the purpose of illustration though it will be evident for those skilled in the art that numerous variations and details of the invention may be made without departing from the invention as described in the claims.

### Examples

Example 1: One could envision a nano-cantilever functionalized with a selectively binding chemical like cyclam, which binds biogenic diamines such as cadaverine selectively. When exposed to cadaverine from degrading meat the functionalized cantilever will bend or change resonance frequency, with this change in the mechanical properties of the cantilever being optically or resistively detectable. The cantilever will be placed in a hand held device; a pen. The tip of the pen will be inserted into the meat and an equilibrium concentration of volatile biogenic amines in the volume between meat and sensor will be established. In a device related ot but not forming part of the invention, this concentration is thus measured by the described technology and the value indicative of the degradation state established from correlation with database values, is shown at a suitable interface and sent to a computer and a remote sever where the data can be further characterised, stored and subsequently accessed. The measured value together with information on further storage conditions will be compiled in pre-determined algorithms, which reflect the degradation state of different kinds of meat at different storage conditions, and from this comparison to known data a correct prediction of remaining shelf-life can be determined and thus prevent food poisoning incidents and the potential detrimental impact thereof; avoiding unnecessary food waste and the associated economic and environmental impacts.

Example 2: One could also envision functionalized cantilever bridges coated with a selective binder with a high affinity towards trimethylamine and samples of vaginal fluids can thus be tested with a test-kit for medical conditions such as bacterial vaginosis. In this test-kit the functionalized cantilever bridge could situated in a disposable cartridge with an adaptable lock-system to the read-out apparatus.

Example 3: As meat is processed to other products the quality is tested in the production line via automatic monitoring by a coated cantilever in a microfluidic device, measuring spermine, tyramine and histamine levels of the liquid associated with the process.

Example 4: During the transportation of fish and seafood an apparatus in the transportation packaging containing the embodied technology for determining volatile biogenic amines will, in real time transmit the condition of the fish to a central server which can be remotely accessed.

Example 5: Since biogenic amines are indicative of organic decomposition, the concentration of these compounds in water samples would indicate a pollution source and determine the health risk associated with drinking the water. This could be accomplished by measuring the concentration of these heat resistant amines after boiling of the majority of water and utilize the technology disclosed in this present invention.

## Claims

1. A device for determining freshness and forecasting expiration dates for meat and fish wherein the device comprises at least one sensing device for detection of biogenic amines, which sensing device comprises at least a nano- or a micro-mechanical sensing unit, **characterised in** the device comprises at least one database, which database comprises experimentally obtained values of biogenic amine concentrations for at least one kind of meat or fish for at least one storage condition say temperature, where the database further comprises at least one algorithm for correlation of the concentration of biogenic amines detected by the sensing device with database values for prediction of expiration, and where the device is configured to forecast expiration dates for meat and fish using said database and said algorithm.

2. Sensing device according to claim 1, **characterised in that** the sensing unit comprises a cantilever with a surface, where at least part of the surface comprises a coating, which coating is a molecular imprinted polymer and where the sensing device further comprises a detecting unit.

3. Sensing device according to any of the claims 1 to 2, **characterised in that** the detecting unit at least comprises an optical detector or an electrical detector.

4. Sensing device according to claim 3, **characterised in that** the optical detector comprises a laser diode and a photodiode facing the cantilever from the same side and with an angle between the laser diode and the photodiode, where the angle is less than 180°.

5. Sensing device according to any of the claims 1 to 4, **characterised in that** the sensing device is a handheld sensing device.

6. Sensing device according to claim 5, **characterised in that** the handheld device is a pen further comprising a display.

7. Sensing device according to any of the claims 1 to 6, **characterised in that** the sensing device further comprises a transmission computer comprising means for storing, processing and accessing data from the sensing device.

8. The sensing device according to one of the claims 1-7, **characterised in that** the sensing device is adapted to monitor food quality.

9. Method of using a sensing device as disclosed in claims 2 to 8, for determining freshness and forecasting expiration dates for meat and fish, which method activates at least a nano- or a micro-mechanical sensing unit, **characterised in that** the method stores the measured data in at least one database, which database comprises experimentally obtained values of biogenic amine concentrations for at least one kind of meat or fish for at least one storage condition say temperature, where the database further comprises at least one algorithm for correlation of the concentration of biogenic amines detected by the sensing device with database values for prediction of expiration.

10. Method according to claim 9, **characterised in that** the method further comprises at least the following steps:
A) Biogenic amine molecules interacts with the cantilever coating,
B) The cantilever bend,
C) The bending is measured,
D) The measured data is displayed at the sensing device and/or the measured data is wirelessly transmitted to a computer.

11. Method of using a sensing device according to claim 10, **characterised in that** the method further comprises at least the following steps after D):
E) the measure is optically by measuring the change in deflection angle of a laser beam from the laser diode sent at the cantilever and reflected to the photodiode.

12. Method of using a sensing device according to any of the claims 9-11, **characterised in that** the method further comprises at least the following step after E):
F) the measured data is stored and processed on the computer comprising means for storing, processing and accessing data, to determine remaining shelf life of meat and fish.

13. Method of using a sensing device according to claim 10 or 12, **characterised in that** the method further comprises at least the following step after F):
G) data is forwarded via the internet to e.g. a database for storage and processing and for remote access via the internet.

## Patentansprüche

1. Vorrichtung zum Bestimmen der Frische und zum Vorhersagen der Verfallsdaten von Fleisch und Fisch, wobei die Vorrichtung mindestens eine Sensorvorrichtung zum Nachweisen biogener Amine umfasst, wobei die Sensorvorrichtung mindestens eine nano- oder mikromechanische Sensoreinheit umfasst, **dadurch gekennzeichnet, dass** die Vorrichtung mindestens eine Datenbank umfasst, wobei die Datenbank experimentell erhaltene Werte von biogenen Aminkonzentrationen für mindestens eine Art von Fleisch oder Fisch für mindestens eine Lagerungsbedingung, z. B. die Temperatur, umfasst, wobei die Datenbank weiter mindestens einen Algorithmus zur Korrelation der Konzentration von durch die Sensorvorrichtung nachgewiesen biogenen Aminen mit Datenbankwerten zur Vorhersage des Verfalls umfasst, und wobei die Vorrichtung konfiguriert ist, um Verfallsdaten für Fleisch und Fisch unter Verwendung der Datenbank und des Algorithmus vorherzusagen.

2. Sensorvorrichtung nach Anspruch 1, **dadurch gekennzeichnet, dass** die Sensoreinheit einen Träger mit einer Oberfläche umfasst, wobei mindestens ein Teil der Oberfläche eine Beschichtung umfasst, die ein molekular geprägtes Polymer ist, und wobei die Sensorvorrichtung weiter eine Detektoreinheit umfasst.

3. Sensorvorrichtung nach einem der Ansprüche 1 bis 2, **dadurch gekennzeichnet, dass** die Detektoreinheit zumindest einen optischen Detektor oder einen elektrischen Detektor umfasst.

4. Sensorvorrichtung nach Anspruch 3, **dadurch gekennzeichnet, dass** der optische Detektor eine Laserdiode und eine Fotodiode umfasst, die dem Träger von derselben Seite aus zugewandt sind und einen Winkel zwischen der Laserdiode und der Fotodiode bilden, wobei der Winkel kleiner als 180° ist.

5. Sensorvorrichtung nach einem der Ansprüche 1 bis 4, **dadurch gekennzeichnet, dass** die Sensorvorrichtung eine handgehaltene Sensorvorrichtung ist.

6. Sensorvorrichtung nach Anspruch 5, **dadurch gekennzeichnet, dass** es sich bei der handgehaltenen Vorrichtung um einen Stift handelt, der weiter ein Display umfasst.

7. Sensorvorrichtung nach einem der Ansprüche 1 bis 6, **dadurch gekennzeichnet, dass** die Sensorvorrichtung weiter einen Übertragungscomputer umfasst, der Mittel zum Speichern, Verarbeiten und Zugreifen auf Daten von der Sensorvorrichtung umfasst.

8. Sensorvorrichtung nach einem der Ansprüche 1-7, **dadurch gekennzeichnet, dass** die Sensorvorrichtung zur Überwachung der Lebensmittelqualität geeignet ist.

9. Verfahren zur Verwendung einer Sensorvorrichtung wie sie in einem der Ansprüche 2 bis 8 offenbart ist zum Bestimmen der Frische und zum Vorhersagen der Verfallsdaten von Fleisch und Fisch, wobei das Verfahren mindestens eine nano- oder mikromechanische Sensoreinheit aktiviert, **dadurch gekennzeichnet, dass** das Verfahren die Messdaten in mindestens einer Datenbank speichert, wobei die Datenbank experimentell erhaltene Werte von biogenen Aminkonzentrationen für mindestens eine Fleisch- oder Fischsorte für mindestens eine Lagerungsbedingung, z. B. die Temperatur, umfasst, wobei die Datenbank weiter mindestens einen Algorithmus zur Korrelation der von der Sensorvorrichtung nachgewiesenen Konzentration biogener Amine mit Datenbankwerten zur Vorhersage des Verfalls umfasst.

10. Verfahren nach Anspruch 9, **dadurch gekennzeichnet, dass** das Verfahren weiter mindestens die folgenden Schritte umfasst:
A) Biogene Aminmoleküle interagieren mit der Träger-Beschichtung,
B) Der Träger verbiegt sich,
C) Die Biegung wird gemessen,
D) Die Messdaten werden an der Sensorvorrichtung angezeigt und/oder die Messdaten werden drahtlos an einen Computer übertragen.

11. Verfahren zur Verwendung einer Sensorvorrichtung nach Anspruch 10, **dadurch gekennzeichnet, dass** das Verfahren weiter mindestens die folgenden Schritte nach D) umfasst:
E) die Messung erfolgt optisch durch Messung der Änderung des Ablenkwinkels eines von der Laserdiode an den Träger gesendeten und zu der Photodiode reflektierten Laserstrahls.

12. Verfahren zur Verwendung einer Sensorvorrichtung nach einem der Ansprüche 9-11, **dadurch gekennzeichnet, dass** das Verfahren weiter mindestens den folgenden Schritt nach E) umfasst:
F) die gemessenen Daten werden auf dem Computer gespeichert und verarbeitet, der Mittel zum Speichern, Verarbeiten und Zugreifen auf Daten umfasst, um die verbleibende Haltbarkeit von Fleisch und Fisch zu bestimmen.

13. Verfahren zur Verwendung einer Sensorvorrichtung nach Anspruch 10 oder 12, **dadurch gekennzeichnet, dass** das Verfahren weiter mindestens den folgenden Schritt nach F) umfasst:
G) die Daten werden über das Internet z. B. an eine Datenbank zur Speicherung und Verarbeitung und zum Fernzugriff über das Internet weitergeleitet.

## Revendications

1. Dispositif pour déterminer la fraîcheur et prévoir les dates d'expiration de viande et poisson, dans lequel le dispositif comprend au moins un dispositif capteur pour la détection d'amines biogènes, lequel dispositif capteur comprend au moins une unité de détection nano- ou micromécanique, **caractérisé en ce que** le dispositif comprend au moins une base de données, laquelle base de données comprend des valeurs obtenues expérimentalement de concentrations d'amines biogènes pour au moins un type de viande ou poisson pour au moins une condition de stockage, telle que la température, la base de données comprenant en outre au moins un algorithme destiné à la corrélation de la concentration d'amines biogènes détectées par le dispositif capteur avec des valeurs de base de données pour la prédiction de l'expiration, and le dispositif étant configuré pour prévoir des dates d'expiration de viande et poisson à l'aide de ladite base de données et dudit algorithme.

2. Dispositif capteur selon la revendication 1, **caractérisé en ce que** l'unité de détection comprend un cantilever présentant une surface, au moins une partie de la surface comprenant un revêtement, lequel revêtement est un polymère à empreinte moléculaire et le dispositif capteur comprenant en outre une unité de détection.

3. Dispositif capteur selon l'une quelconque des revendications 1 à 2, **caractérisé en ce que** l'unité de détection comprend au moins un détecteur optique ou un détecteur électrique.

4. Dispositif capteur selon la revendication 3, **caractérisé en ce que** le détecteur optique comprend une diode laser et une photodiode faisant face au cantilever depuis le même côté et avec un angle entre la diode laser et la photodiode, l'angle étant inférieur à 180°.

5. Dispositif capteur selon l'une quelconque des revendications 1 à 4, **caractérisé en ce que** le dispositif capteur est un dispositif capteur portatif.

6. Dispositif capteur selon la revendication 5, **caractérisé en ce que** le dispositif portatif est un stylo comprenant en outre un affichage.

7. Dispositif capteur selon l'une quelconque des revendications 1 à 6, **caractérisé en ce que** le dispositif capteur comprend en outre un ordinateur à transmission comprenant des moyens pour la mémorisation de, le traitement de et l'accès à des données provenant du dispositif capteur.

8. Dispositif capteur selon l'une des revendications 1-7, **caractérisé en ce que** le dispositif capteur est adapté à surveiller la qualité d'aliments.

9. Procédé d'utilisation d'un dispositif capteur tel que divulgué dans les revendications 2 à 8, pour déterminer la fraîcheur et prévoir les dates d'expiration de viande et poisson, lequel procédé active au moins une unité de détection nano-ou micromécanique, **caractérisé en ce que** le procédé mémorise dans au moins une base de données les données mesurées, laquelle base de données comprend des valeurs obtenues expérimentalement de concentrations d'amines biogènes pour au moins un type de viande ou poisson pour au moins une condition de stockage, telle que la température, la base de données comprenant en outre au moins un algorithme destiné à la corrélation de la concentration d'amines biogènes détectées par le dispositif capteur avec des valeurs de base de données pour la prédiction de l'expiration.

10. Procédé selon la revendication 9, **caractérisé en ce que** le procédé comprend en outre au moins les étapes suivantes :
A) des molécules d'amines biogènes interagissent avec le revêtement du cantilever,
B) le cantilever fléchit,
C) le fléchissement est mesuré,
D) les données mesurées sont affichées au niveau du dispositif capteur et/ou les données sont transmises sans fil à un ordinateur.

11. Procédé d'utilisation d'un dispositif capteur selon la revendication 10, **caractérisé en ce que** le procédé comprend en outre au moins l'étape suivante après D) :
E) la mesure est effectuée optiquement par mesure de la modification de l'angle de déflexion d'un faisceau laser provenant de la diode laser, envoyé sur le cantilever et réfléchi vers la photodiode.

12. Procédé d'utilisation d'un dispositif capteur selon l'une quelconque des revendications 9-11, **caractérisé en ce que** le procédé comprend en outre au moins l'étape suivante après E) :
F) les données mesurées sont mémorisées et traitées sur l'ordinateur comprenant des moyens pour la mémorisation de, le traitement de et l'accès à des données, afin de déterminer la durée restante de conservation de viande et poisson.

13. Procédé d'utilisation d'un dispositif capteur selon la revendication 10 ou 12, **caractérisé en ce que** le procédé comprend en outre au moins l'étape suivante après F) :
G) les données sont transmises via l'internet à par exemple une base de données pour mémorisation et traitement et pour accès à distance via l'internet.
